# EUROPEAN PATENT SPECIFICATION

(11) **EP 3 400 241 B1**
(45) Date of publication and mention of the grant of the patent: **22.07.2020**
(21) Application number: 17702444.5
(22) Date of filing: 05.01.2017
(51) Int. Cl.: C07K 16/24

(54) **MODULATION OF AFUCOSYLATED SPECIES IN A MONOCLONAL ANTIBODY COMPOSITION**
MODULATION VON AFUCOSYLIERTEN SPEZIES IN EINER MONOKLONALEN ANTIKÖRPERZUSAMMENSETZUNG
MODULATION D'ESPÈCES AFUCOSYLÉES DANS UNE COMPOSITION D'ANTICORPS MONOCLONAL

(30) Priority: 06.01.2016 US 201662275384 P
(43) Date of publication of application: 14.11.2018
(73) Proprietor: Outlook Therapeutics, Inc., Cranbury, NJ 08512 (US)
(72) Inventor: SANTORO, Marc, Cranbury, NJ 08512 (US); JOSE, Kevin, John, Cranbury, NJ 08512 (US); MADABHUSHI, Sri, Cranbury, NJ 08512 (US); GANGLOFF, Scott, Cranbury, NJ 08512 (US)
(74) Representative: Cooley (UK) LLP
(86) International application number: PCT/US2017/012349
(87) International publication number: WO 2017/120347

(56) References cited:
- WO-A1-2010/141855
- WO-A1-2012/041768
- WO-A1-2013/114165
- WO-A1-2014/055370
- WO-A1-2015/140700
- KANDA ET AL: "Establishment of a GDP-mannose 4,6-dehydratase (GMD) knockout host cell line: A new strategy for generating completely non-fucosylated recombinant therapeutics", JOURNAL OF BIOTECHNOLOGY, ELSEVIER, AMSTERDAM, NL, vol. 130, no. 3, 19 June 2007 (2007-06-19) , pages 300-310, XP022119693, ISSN: 0168-1656, DOI: 10.1016/J.JBIOTEC.2007.04.025
- IMAI-NISHIYA HARUE ET AL: "Double knockdown of Î+-1,6-fucosyltransferase (FUT8) and GDP-mannose 4,6-dehydratase (GMD) in antibody-producing cells: a new strategy for generating fully non-fucosylated therapeutic antibodies with enhanced ADCC", BMC BIOTECHNOLOGY, BIOMED CENTRAL LTD. LONDON, GB, vol. 7, no. 1, 30 November 2007 (2007-11-30), page 84, XP021035667, ISSN: 1472-6750
- VON HORSTEN H H ET AL: "Production of non-fucosylated antibodies by co-expression of heterologous GDP-6-deoxy-D-lyxo-4-hexulose reductase", GLYCOBIOLOGY, OXFORD UNIVERSITY PRESS, US, vol. 20, no. 12, 1 December 2010 (2010-12-01), pages 1607-1618, XP002612500, ISSN: 0959-6658, DOI: 10.1093/GLYCOB/CWQ109 [retrieved on 2010-07-15]
- ZHOU QUIN ET AL: "Development of a simple and rapid method for producing non-fucosylated oligomannose containing antibodies with increased effector function", BIOTECHNOLOGY AND BIOENGINEERING, WILEY ETC, vol. 99, no. 3, 1 February 2008 (2008-02-01), pages 652-665, XP009137262, ISSN: 0006-3592, DOI: 10.1002/BIT.21598
- ANA RITA COSTA ET AL: "The impact of microcarrier culture optimization on the glycosylation profile of a monoclonal antibody", SPRINGERPLUS, vol. 2, no. 1, 28 January 2013 (2013-01-28), page 25, XP55345245, DE ISSN: 2193-1801, DOI: 10.1186/2193-1801-2-25
- SATOH MITSUO ET AL: "NON-FUCOSYLATED THERAPEUTIC ANTIBODIES AS NEXT-GENERATION THERAPEUTIC ANTIBODIES", EXPERT OPINION ON BIOLOGICAL THERAPY, INFORMA HEALTHCARE, ASHLEY, LONDON; GB, vol. 6, no. 11, 1 November 2006 (2006-11-01), pages 1161-1173, XP008078583, ISSN: 1471-2598, DOI: 10.1517/14712598.6.11.1161
- KATSUHIRO MORI ET AL: "Non-fucosylated therapeutic antibodies: the next generation of therapeutic antibodies", CYTOTECHNOLOGY, KLUWER ACADEMIC PUBLISHERS, DO, vol. 55, no. 2-3, 31 October 2007 (2007-10-31), pages 109-114, XP019550382, ISSN: 1573-0778, DOI: 10.1007/S10616-007-9103-2
- YAMANE-OHNUKI NAOKO ET AL: "Production of therapeutic antibodies with controlled fucosylation", MABS, LANDES BIOSCIENCE, US, vol. 1, no. 3, 1 May 2009 (2009-05-01), pages 230-236, XP002630025, ISSN: 1942-0870
- JOHN G. ALLEN ET AL: "Facile Modulation of Antibody Fucosylation with Small Molecule Fucostatin Inhibitors and Cocrystal Structure with GDP-Mannose 4,6-Dehydratase", ACS CHEMICAL BIOLOGY, vol. 11, no. 10, 21 October 2016 (2016-10-21), pages 2734-2743, XP055352875, US ISSN: 1554-8929, DOI: 10.1021/acschembio.6b00460

## Description

### FIELD OF THE INVENTION

The disclosure relates generally to the field of protein biochemistry. More particularly, the invention relates to a nutrient feed scheme for recombinant antibody-expressing cells in a bioreactor, which both substantially reduces undesirable isoforms of the antibody being expressed, and is capable of modulating the level of afucosylated species of the antibody being expressed.

### BACKGROUND OF THE INVENTION

Various publications, including patents, published applications, accession numbers, technical articles and scholarly articles are cited throughout the specification.

As part of the Biologics Price Competition and Innovation Act (BPCIA), a biological drug product (produced in or derived from living organisms) may be demonstrated to be "biosimilar" if data show that, among other things, the product is "highly similar" to an already-approved biological product. The biosimilar product should retain at least the biologic function and treatment efficacy of the U.S. Food and Drug Agency-approved biological product.

Monoclonal antibodies (mAbs) may be used as therapeutic proteins. Purified monoclonal antibodies are most often present in a complex heterogeneous mixture based on chemical modifications of selected amino acids sites that range from subtle to significant. Understanding the impact of these modifications is of considerable importance in the biotechnology field. Monoclonal antibodies have charge heterogeneity that optimizes the balance of gaining favorable electrostatic interactions and determines their structure, stability, binding affinity, chemical properties and, hence, their biological activity.

Consistency of the drug substance and product along with a maximized shelf life are of paramount importance to drug developers and manufacturers. Short shelf life of drug substance and product usually translate to manufacturing challenges and high costs of production by manufacturers. During the cell culture or fermentation process antibodies and proteins may undergo phenomena known as post-translational modifications. These modifications contribute to several forms of heterogeneity seen in therapeutic proteins. Additionally, there are forms of heterogeneity that occur during the manufacture caused by stresses imparted during the process such as size and charge that can occur due to enzymatic processes or spontaneous degradation and modifications. mAbs undergo chemical modification via several different mechanisms, including oxidation, deamidation, glycation, isomerization and fragmentation, that result in the formation of various charge variants and heterogeneity.

Chemical and enzymatic modifications such as deamidation, and sialylation, result in an increase in the net negative charge on mAbs and cause a decrease in pI values, thereby leading to formation of acidic variants. C-terminal lysine cleavage results in the loss of net positive charge and leads to acidic variant formation. Another mechanism for generating acidic variants is the formation of various types of covalent adducts, e.g., glycation, where glucose or lactose can react with the primary amine of a lysine or arginine residue during manufacturing in glucose-rich culture media or during storage if a reducing sugar is present in the formulation. Formation of the basic variants can result from the presence of one or more C-terminal lysines or proline amidation, succinimide formation, amino acid oxidation or removal of sialic acid, which introduce additional positive charges or removal of negative charges; both types of modifications cause an increase in pI values.

Although there is substantial knowledge and experience with the degradation pathways that are active during production and formulation, a current challenge is to understand how the heterogeneity described above may affect efficacy, potency, immunogenicity and clearance. Little is known about the effects of charge on the PK of subcutaneously (SC) administered mAbs. Passage through the interstitium to the vascular or lymphatic capillaries can present a barrier to efficient drug absorption after SC administration. Interstitial diffusion of mAbs is likely to be influenced by their charge and their electrostatic interactions with negatively charged constituents of the interstitial area underlying the dermis of the skin.

Recently, the growth and interest in the development of biosimilars has presented several unique challenges to the production of biotherapeutics such as mAbs. The development of innovative molecules allows latitude to define the product quality attributes (PQAs) and, ultimately, the critical quality attributes (CQAs) of a mAb during the natural course of the development process. This paradigm, in turn, permits the implementation of a potentially robust production platform capable of handling the mAbs that a pipeline of candidates may produce with minimal optimization.

The development of biosimilar molecules, by contrast, imposes the confines of a predefined (by the reference product) set of product quality attribute ranges. The impact on process development is that the latitude that a platform process may afford may be significantly reduced by the requirement to fit within a defined range for multiple PQAs. This is especially true for those attributes that are known to be or could potentially be biologically relevant such as the charge variants described above. Purification or reduction of such heterogeneity so as to achieve a more homogenous population poses a significant challenge to process developers. The differences in the species that make up the heterogeneous population of charge variants are often quite subtle and similar in their characteristics to the primary mAb population of interest. Consequently, these unwanted variants are difficult to separate effectively while maintaining a reasonable mAb recovery. There is a need to minimize these unwanted variants toward a more homogenous population of biosimilar mAbs.
WO 2010/141855 A1 (Momenta Pharmaceuticals, Inc.; 9 December 2010) discusses methods and materials for monitoring and regulating the glycosylation of glycoproteins that are recombinantly produced from cells, such as the level of fucosylation.

### SUMMARY OF THE INVENTION

The disclosure features methods for enhancing or reducing the levels of afucosylated species of a monoclonal antibody recombinantly expressed in a bioreactor. In addition, the disclosure also features methods for reducing the levels of high molecular weight species, acidic species, and fragments of a monoclonal antibody recombinantly expressed in a bioreactor.

In an aspect of the invention, provided herein are methods for reducing afucosylated species of a monoclonal antibody recombinantly expressed in a bioreactor, by culturing recombinant cells that express the monoclonal antibody in a bioreactor, and beginning on the first, second, third, fourth, or fifth day of the culture, and continuing every day until the conclusion of the culture, infusing the culture with from about 0.5 g/L to about 5 g/L of fucose such that afucosylated species of the monoclonal antibody expressed by the cells are reduced.

Also provided are methods for reducing afucosylated species of a monoclonal antibody recombinantly expressed in a bioreactor, by culturing recombinant cells that express the monoclonal antibody in a bioreactor, beginning on the first, second, third, or fourth day of the culture, and continuing every other day until the conclusion of the culture, infusing the culture with from about 0.5 g/L to about 5 g/L of fucose such that afucosylated species of the monoclonal antibody expressed by the cells are reduced.

According to the methods of the disclosure, the monoclonal antibody may specifically bind to tumor necrosis factor (TNF) alpha.

Likewise, according to methods of the disclosure, the recombinant cells may include mammalian cells *(e.g.,* Chinese Hamster Ovary cells, HEK293 cells, Sp2/0 cells, or any other suitable cells that may be grown in a bioreactor for monoclonal antibody expression).

In any of the methods disclosed herein, the culture can be infused with from about 1 g/L to about 5 g/L of fucose, from about 1 g/L to about 4 g/L of fucose, from about 1 g/L to about 3 g/L of fucose, from about 1 g/L to about 2 g/L of fucose, and/or from about 2 g/L to about 4 g/L of fucose.

In some embodiments, the fucose is infused in a bolus.

In some embodiments of the methods of the disclosure, the culture is a continuous-feed culture in which the culture is further infused with a feed media continuously over a twenty four hour period beginning on the second, third, fourth, or fifth day of the culture, and continuing every day until the conclusion of the culture. In other embodiments of the methods of the disclosure, the culture is an extended-feed culture in which the culture is further infused with a feed media continuously over a period of from about eighteen to about twenty hours beginning on the second, third, fourth, or fifth day of the culture, and continuing every day until the conclusion of the culture.

Exemplary afucosylated species include the GO glycan, G1a glycan, G1b glycan, G2 glycan, Man 3 glycan, Man 4 glycan, Man 5 glycan, Man 6 glycan, Man 7 glycan, Man 8 glycan, and/or Man 9 glycan, or any combinations thereof. In one non-limiting preferred embodiment, the afucosylated species is the GO glycan.

According to the methods of the disclosure, the afucosylated species of the monoclonal antibody are reduced to about 10% or less of the total amount of monoclonal antibody expressed by the cells; are reduced to from about 2% to about 10% of the total amount of monoclonal antibody expressed by the cells; are reduced to about 6% or less of the total amount of monoclonal antibody expressed by the cells; and or are reduced to from about 3% to about 7% of the total amount of monoclonal antibody expressed by the cells.

In the methods of the disclosure, the recombinant cells that express the monoclonal antibody can be cultured in a bioreactor for at least twelve days.

Methods are also provided for enhancing afucosylated species by culturing recombinant cells that express the monoclonal antibody in a bioreactor, then beginning on the first, second, third, fourth, or fifth day of the culture, and continuing every day until the conclusion of the culture, infusing the culture with a feed media continuously over a twenty four hour period sufficiently to enhance the level of afucosylated species of the monoclonal antibody. Likewise, methods are provided for enhancing afucosylated species by culturing recombinant cells that express the monoclonal antibody in a bioreactor, then beginning on the first, second, third, fourth, or fifth day of the culture, and continuing every day until the conclusion of the culture, infusing the culture with a feed media continuously for a period of from about eighteen to about twenty hours sufficiently to enhance the level of afucosylated species of the monoclonal antibody.

Such continuous or extended feeding of the cell culture may enhance one or more of monoclonal antibody species containing one or more of the GO glycan, G1a glycan, G1b glycan, G2 glycan, Man 3 glycan, Man 4 glycan, Man 5 glycan, Man 6 glycan, Man 7 glycan, Man 8 glycan, or Man 9 glycan, including any combination thereof. In one example, continuous or extended feeding of the cell culture may enhance one or more monoclonal antibody species including the GO glycan.

Continuous or extended feeding of the cell culture may enhance the afucosylated species of the monoclonal antibody to from 1% to about 10% of the total amount of monoclonal antibody expressed by the cells, including to from about 1% to about 5% of the total amount of monoclonal antibody expressed by the cells, from about 5% to about 10% of the total amount of monoclonal antibody expressed by the cells, from about 7% to about 10% of the total amount of monoclonal antibody expressed by the cells, to from about 8% to about 10% of the total amount of monoclonal antibody expressed by the cells, or to from about 8.5% to about 9.5% of the total amount of monoclonal antibody expressed by the cells. Continuous or extended feeding of the cell culture may enhance the GO afucosylated species of the monoclonal antibody to from about 6% to about 9% of the total amount of monoclonal antibody expressed by the cells, including to from about 6% to about 8% of the total amount of monoclonal antibody expressed by the cells, from about 6% to about 7% of the total amount of monoclonal antibody expressed by the cells, or from about 7% to about 8% of the total amount of monoclonal antibody expressed by the cells.

Methods are also provided for reducing the levels of high molecular weight species, acidic species, and fragments of a monoclonal antibody by culturing recombinant cells that express the monoclonal antibody in a bioreactor, then beginning on the first, second, third, fourth, or fifth day of the culture, and continuing every day until the conclusion of the culture, infusing the culture with a feed media continuously over a twenty four hour period sufficiently to reduce one or more of the high molecular weight species, acidic charge species, and fragments of the monoclonal antibody. Likewise, methods are also provided for reducing the levels of high molecular weight species, acidic species, and fragments of a monoclonal antibody by culturing recombinant cells that express the monoclonal antibody in a bioreactor, then beginning on the first, second, third, fourth, or fifth day of the culture, and continuing every day until the conclusion of the culture, infusing the culture with a feed media continuously for a period of from about eighteen to about twenty hours sufficiently to reduce one or more of the high molecular weight species, acidic charge species, and fragments of the monoclonal antibody. Such continuous or extended feeding of the cell culture may reduce high molecular weight species, acidic species, and fragments of the monoclonal antibody relative to the levels of high molecular weight species, acidic species, and fragments of the antibody expressed by a bolus-fed culture.

By feeding the cell culture according to a continuous or extended feeding scheme, the high molecular weight species of the monoclonal antibody are reduced to about 5% or less of the total amount of monoclonal antibody expressed by the cells. By feeding the cell culture according to a continuous or extended feeding scheme, the acidic charge species of the monoclonal antibody are reduced to about 5% or less of the total amount of monoclonal antibody expressed by the cells. By feeding the cell culture according to a continuous or extended feeding scheme, fragments of the monoclonal antibody are reduced to about 5% or less of the total amount of monoclonal antibody expressed by the cells.

Any of these methods described herein can be used to reduce one or more of monoclonal antibody fragments including constant region, variable region, heavy chain, light chain, heavy chain variable region, light chain variable region, heavy chain CDR1, heavy chain CDR2, heavy chain CDR3, light chain CDR1, light chain CDR2, and/or light chain CDR3. In some examples, the methods reduce all of these fragments. However, as each of these fragments varies significantly in size, monoclonal antibody fragments might be differentially excluded.

In addition, as some monoclonal antibody fragments are "functionally active" (could bind an antigen) while others are not, the methods described herein can be used to reduce the non-functionally active monoclonal antibody fragments. Likewise, some fragments of monoclonal antibodies (*i.e.,* pairs of heavy chains and light chains that scFvs and VHH) can be used therapeutically. Thus, in some examples, the methods described herein can be used to isolate variant antibody compositions by reducing the presence of non-therapeutically active monoclonal antibodies fragments in the compositions.

In any of the foregoing methods, or any methods described or exemplified herein, the monoclonal antibody may be an antibody that specifically binds to tumor necrosis factor (TNF) alpha.

Likewise, in any of the foregoing methods, or any methods described or exemplified herein, the culture is a culture of mammalian cells that express the antibody. The cells may be CHO cells, HEK293 cells, NSO cells, Sp2/0 cells, or any other suitable cells that may be grown in a bioreactor for monoclonal antibody expression.

Any of the aspects and embodiments described herein can be combined with any other aspect or embodiment as disclosed here in the Summary of the Invention, in the Drawings, and/or in the Detailed Description of the Invention, including the below specific, non-limiting, examples/embodiments of the present invention.

Unless otherwise defined, all technical and scientific terms used herein have the same meaning as commonly understood by one of ordinary skill in the art to which this application belongs. In the specification, the singular forms also include the plural unless the context clearly dictates otherwise.

Although methods and materials similar to or equivalent to those described herein can be used in the practice and testing of the application, suitable methods and materials are described below.

The references cited herein are not admitted to be prior art to the claimed application. In the case of conflict, the present specification, including definitions, will control.

Other features and advantages of the application will become apparent from the following detailed description in conjunction with the examples.

### BRIEF DESCRIPTION OF THE DRAWINGS

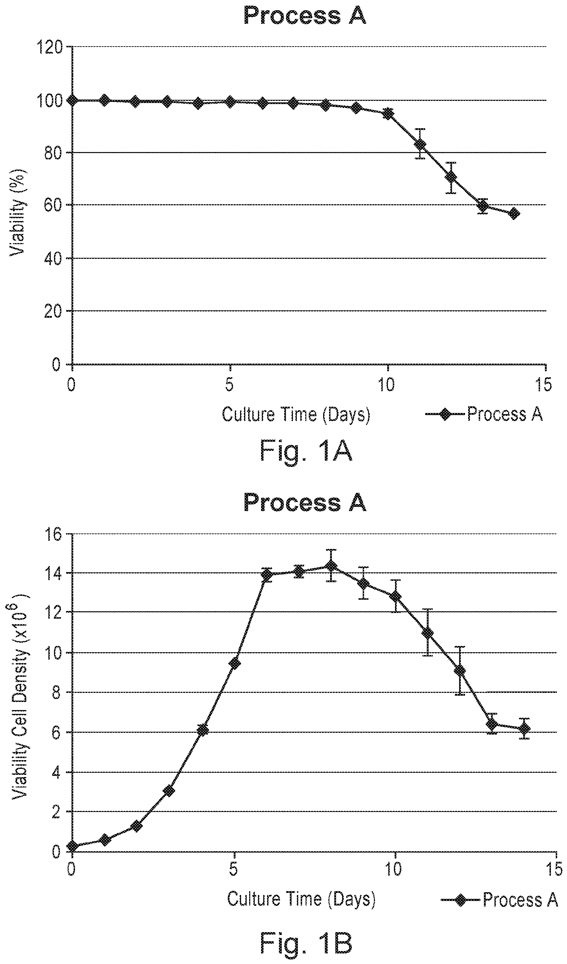

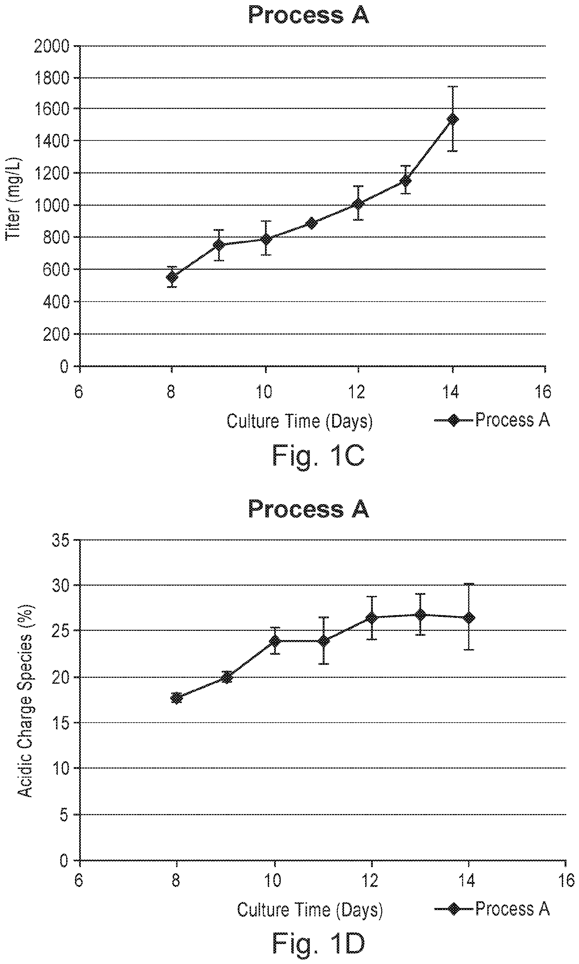

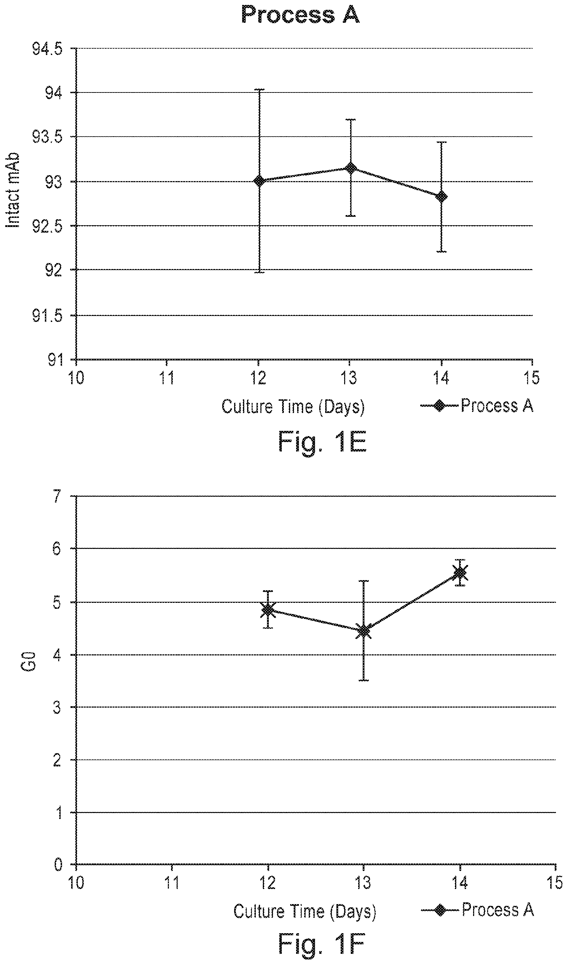

Figures 1A through IF show how Process A affects various aspects of the cell culture and antibody preparation. Figure 1A shows how Process A affects viable cell density over the culture period. Figure IB shows how Process A affects cell viability over the culture period. Figure 1C shows how Process A affects cell titer over the culture period. Figure ID shows how Process A affects acid charge species levels over the culture period. Figure IE shows how Process A affects levels of the intact monoclonal antibody (the desired molecule) over the culture period. Figure IF shows how Process A affects levels of GO afucosylated species over the culture period.

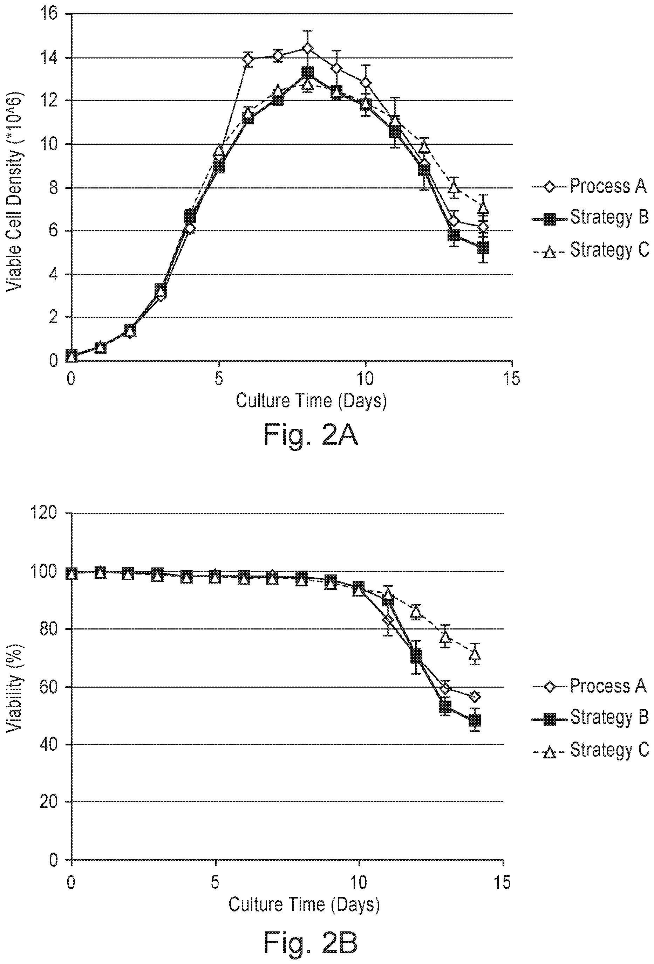

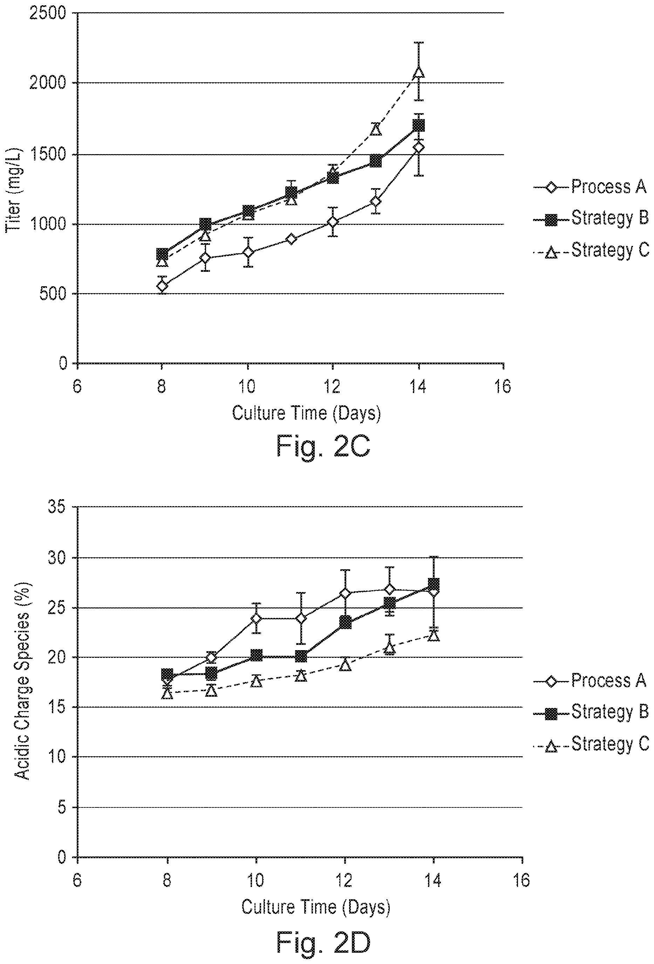

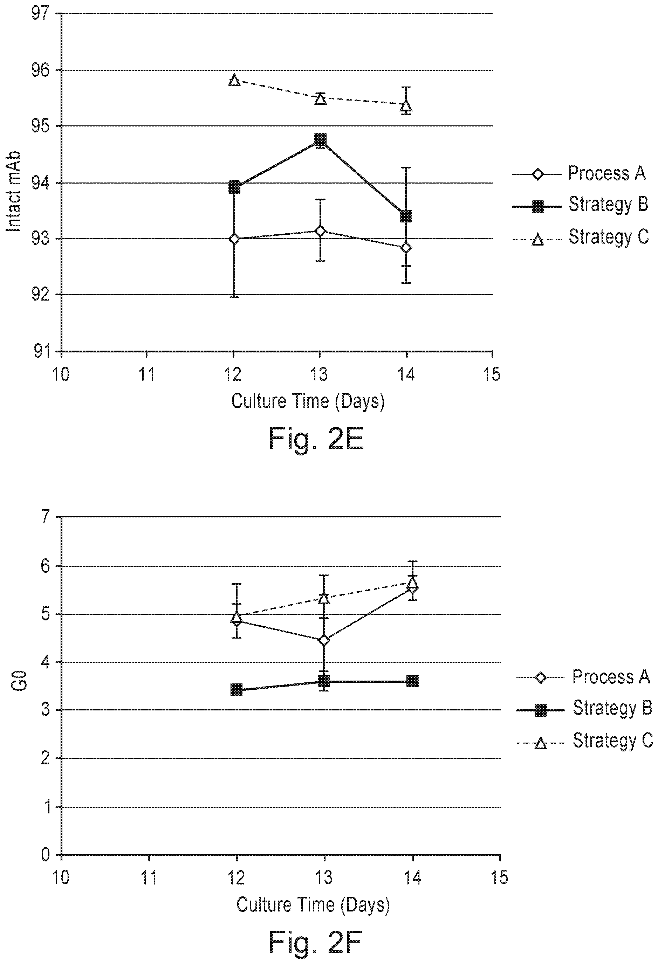

Figures 2A through 2F compare Processes A (diamond), B (square), and C (triangle) and how each affects various aspects of the cell culture and antibody preparation. Figure 2A compares how Processes A, B, and C affect viable cell density over the culture period. Figure 2B compares how Processes A, B, and C affect cell viability over the culture period. Figure 2C compares how Processes A, B, and C affect cell titer over the culture period. Figure 2D compares how Processes A, B, and C affect acid charge species levels over the culture period. Figure 2E compares how Processes A, B, and C affect levels of the intact monoclonal antibody (the desired molecule) over the culture period. Figure 2F compares how Processes A, B, and C affect levels of GO afucosylated species over the culture period.
Figure 3 shows the effect of fucose infusion on levels of GO afucosylated species. These data compare a two day fucose infusion (days 4 and 6) and a 4 day fucose infusion (days 4, 6, 8, and 10), with a continuous (daily) fucose infusion), and no fucose infusion.
Figure 4 shows the effect of fucose infusion on the total levels of afucosylated species. These data compare a two day fucose infusion (days 4 and 6) and a 4 day fucose infusion (days 4, 6, 8, and 10), with a continuous (daily) fucose infusion), and no fucose infusion.

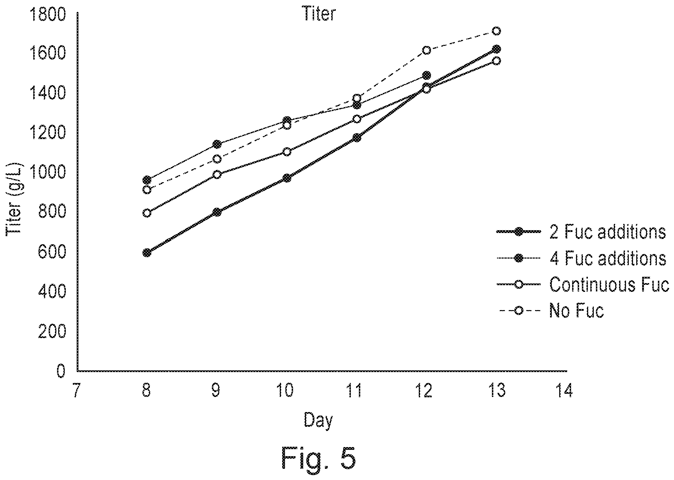

Figure 5 shows the effect of fucose infusion on antibody titer. These data compare a two day fucose infusion (days 4 and 6) and a 4 day fucose infusion (days 4, 6, 8, and 10), with a continuous (daily) fucose infusion), and no fucose infusion.

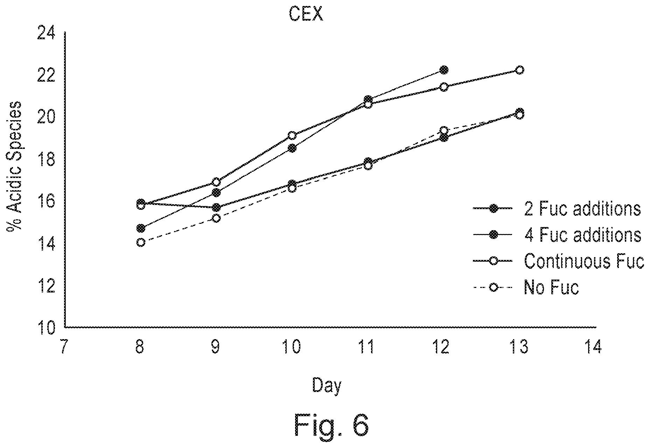

Figure 6 shows the effect of fucose infusion on levels of acidic species. These data compare a two day fucose infusion (days 4 and 6) and a 4 day fucose infusion (days 4, 6, 8, and 10), with a continuous (daily) fucose infusion), and no fucose infusion.

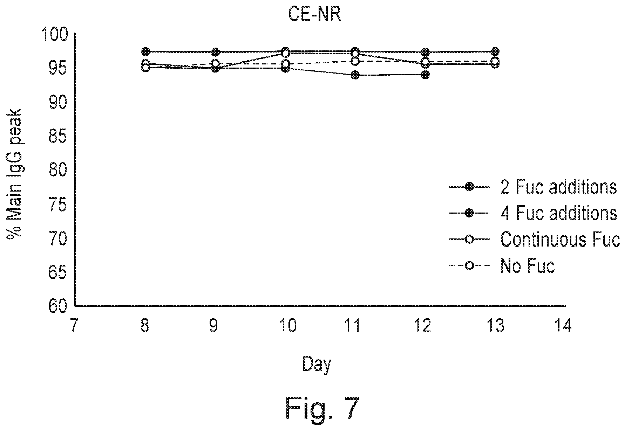

Figure 7 shows the effect of fucose infusion on levels of intact antibody. These data compare a two day fucose infusion (days 4 and 6) and a 4 day fucose infusion (days 4, 6, 8, and 10), with a continuous (daily) fucose infusion), and no fucose infusion.
Figure 8 shows representations of afucosylated glycans.

### DETAILED DESCRIPTION OF THE INVENTION

Various terms relating to aspects of the present invention are used throughout the specification and claims. Such terms are to be given their ordinary meaning in the art, unless otherwise indicated. Other specifically defined terms are to be construed in a manner consistent with the definition provided herein.

As used herein, the singular forms "a," "an," and "the" include plural referents unless expressly stated otherwise.

As used herein, the terms "comprising," "having," and "including" encompass the more restrictive terms "consisting essentially of' and "consisting of."

The terms subject and patient are used interchangeably, and include any animal. Subjects include mammals, including companion and farm mammals, as well as rodents, including mice, rabbits, and rats, and other rodents. In some examples, non-human primates are preferred subjects, and human beings are highly preferred subjects.

As used herein, the term "high molecular weight species of the monoclonal antibody" refers to antibody aggregation, including, for example, antibody dimer, trimer, and multimer formation.

As used herein, the term "acidic charge species of the monoclonal antibody" refers to post-translation modifications of the antibody that cause an acidic charge variant, as compared to the main species (with no modifications) and the basic charge variant.

As used herein, "fragments" of monoclonal antibodies include, but are not limited to constant region, variable region, heavy chain, light chain, heavy chain variable region, light chain variable region, heavy chain CDR1, heavy chain CDR2, heavy chain CDR3, light chain CDR1, light chain CDR2, and/or light chain CDR3. "Functionally active" fragments can include any monoclonal antibody fragments that are capable of binding an antigen.

As used herein, the term "afucosylated monoclonal antibodies" refers to monoclonal antibodies engineered so that the oligosaccharides in the Fc region of the antibody do not have any fucose sugar units. When antibodies are afucosylated, the effect is to increase antibody-dependent cellular cytotoxicity (ADCC).

Typical bioreactor (e.g., fermentation) cell cultures are initiated with a basal medium, with nutrients periodically infused after culture initiation and until the completion of the culture. This infusion is generally of a feed medium, and sustains the cell culture during the protein expression phase. For the most part, feed medium infusion is carried out via a bolus infusion, with concentrated feed medium quickly added into the cell culture at set time points, usually once per day.

When the bioreactor culture is used to express monoclonal antibodies, the monoclonal antibody preparation includes undesirable impurities, which include charge variant species of the antibody, fragments of the antibody, aggregates of the antibody, and other variant species. These impurities, being structurally related to the desired monoclonal antibody, are generally difficult to remove during subsequent purification processes such as chromatography, because the purification processes are insufficiently sensitive to fully discriminate between the desired antibody product and the undesired species, fragments, etc.

The inclusion of variant species, fragments, aggregates, and other undesired variations of the desired antibodies has implications for ultimate potency of the antibody preparation. In the case of afucosylated variants of the antibody, for example, such afucosylated species of the antibody have implications for immune effector function, such as antibody-dependent cell-mediated cytotoxicity (ADCC), which is effectuated, in part, by glycosylation patterns on the antibody Fc and, in particular, fucose levels in the glycan moieties. Accordingly, it is desirable to be able to control antibody species and variants in order to modulate potency and ADCC, among other characteristics. In the case of biosimilar antibody production, it is further desirable to control such species and variants in order that the antibody preparation sufficiently matches the reference product in order to pass regulatory scrutiny and maintain status as a biosimilar product.

It has been observed in accordance with the present disclosure that bioreactor cell culture conditions and, in particular, the feed schedule and type have a direct effect on the levels of particular variant species. It was observed that changing from a bolus feed to a more extended or even to a continuous feed significantly reduced the level of acid charge variants and antibody fragments in the monoclonal antibody preparation expressed in the bioreactor. Additionally, it was observed that such a change in the feed technique also enhanced the health of the cell culture. One consequence of this change, however, was that the level of afucosylated species of the antibody was significantly enhanced.

Although enhanced levels of afucosylated species are desirable in some cases, enhanced levels of afucosylated species are not desirable in other cases. Accordingly, to maintain the benefits of the changed feed scheme but to reduce the level of afucosylated species in antibody preparations where enhanced levels are not desirable, experiments were undertaken to determine how to reduce the afucosylated species without causing any detriment to the beneficial feed scheme or any new detriment to the antibody preparation. It was observed in accordance with such experiments that infusing the bioreactor cell culture with fucose according to a particular infusion schedule was able to achieve a reduction of afucosylated species without enhancing the level of other species or variants of the monoclonal antibody being expressed and without negatively affecting the overall health of the cell culture.

Accordingly, the present disclosure features methods for reducing levels of charge variants, fragments, and aggregates of a monoclonal antibody recombinantly expressed in a bioreactor. The invention features methods for modulating levels of afucosylated species of a monoclonal antibody recombinantly expressed in a bioreactor. The methods may be employed separately or together.

Methods for reducing afucosylated species of a monoclonal antibody recombinantly expressed in a bioreactor generally involve culturing recombinant cells that express the monoclonal antibody in a bioreactor, then infusing the culture with from about 0.5 g/L to about 5 g/L of fucose such that afucosylated species of the monoclonal antibody expressed by the cells are reduced. The infusion of fucose is initiated and coincides with, timing-wise, infusion of the cell culture with feed media. In certain embodiments, it is preferred that the infusion of fucose does not occur in the days prior to the onset of feed media infusion. Fucose and/or feed media infusion may be according to any suitable technique in the art, for example, via a port in communication with the bioreactor cell culture. In the case that the fucose is supplemented in the feed medium, the feed is infused via a peristaltic pump and tubing at the desired rate. If the fucose is added separately (bolus), then it is infused over approximately 2 to 5 minutes.

Any suitable feed media may be infused into the cell culture. Feed media generally are commercially available, and such commercially produced feed media are suitable for use in the methods described or exemplified herein. Feed media may include any media described or exemplified herein.

Infusion of feed media may begin on about day 1, about day 2, about day 3, about day 4, about day 5, about day 6, or about day 7 of a multi-day bioreactor cell culture, and continue thereafter until the conclusion of the cell culture. The cell culture may be at least about 7 days, at least about 10 days, at least about 11 days, at least about 12 days, at least about 13 days, at least about 14 days, at least about 15 days, at least about 16 days, at least about 17 days, at least about 18 days, at least about 19 days, at least about 20 days, at least about 21 days, at least about 22 days, at least about 23 days, at least about 24 days, or more than 24 days. The cell culture can last for from about 10 days to about 21 days, from about 10 days to about 14 days, from about 10 days to about 12 days, or from about 11 days to about 13 days.

Infusion of feed media and infusion of fucose begins on about day three, about day four, or about day five of the culture, and continues every day thereafter until the conclusion of the culture. Infusion of feed media and fucose may begin on about day one or about day two of the culture, and continue every day thereafter until the conclusion of the culture. The infusion of the feed media is according to an extended feed schedule, a continuous feed schedule, or a combination of an extended feed schedule and a continuous feed schedule.

A continuous feed schedule involves infusing the cell culture with feed media continuously over a twenty four hour period. The same amount/concentration of feed media that would ordinarily be infused as a bolus is infused into the culture, but that amount is apportioned to be substantially evenly infused over twenty four hours. A higher amount/concentration of feed media is infused over twenty four hours relative to the amount/concentration that would have been infused per bolus. A lesser amount/concentration of feed media is infused over twenty four hours relative to the amount/concentration that would have been infused per bolus. Thus, under a continuous feed schedule, feed media is infused into the cell culture constantly from the onset of the feed media infusion until the end of the cell culture, or until it is desired to end feed media infusion into the cell culture.

An extended feed schedule involves infusing the cell culture with feed media for a period longer than a bolus infusion, but not over a full twenty four hours. A bolus infusion includes an infusion over a period of from about 5 minutes to about one hour. Generally, a bolus infusion proceeds over a period of from about 5 minutes to about 15 minutes.

An extended feed schedule extends delivery of the feed media into the cell culture over a period of from about six hours to about twenty three hours. An extended feed schedule extends delivery of the feed media into the cell culture over a period of from about eight hours to about eighteen hours, from about twelve hours to about twenty two hours, from about twelve hours to about twenty hours, from about twelve hours to about eighteen hours, from about sixteen hours to about twenty two hours, from about sixteen hours to about twenty hours, from about sixteen hours to about eighteen hours, or from about seventeen hours to about nineteen hours. In some embodiments, an extended feed period of about eighteen hours is preferred. During the balance of time from the end of the extended feed period to the twenty four hour point, no feed media is infused into the cell culture. Thus, under an extended feed schedule, feed media is infused into the cell culture daily over the extended feed period (followed by a break until about the twenty four hour mark) from the onset of the feed media infusion until the end of the cell culture, or until it is desired to end feed media infusion into the cell culture.

Fucose infusion may follow the feed media infusion schedule. Thus, for example, if feed media infusion begins on day four of the culture, fucose infusion also begins on day four of the culture. In some embodiments, it is preferred that fucose infusion does not precede feed media infusion. Fucose infusion may coincide with feed media infusion. For example, when an extended feed schedule is used, fucose is infused at some point during the infusion of the feed media, and not during the period of no feed media infusion.

Fucose infusion can be a bolus infusion and not an extended feed infusion or continuous infusion. Thus, fucose is infused into the cell culture in a concentrated form for a period of from about 5 minutes to about an hour, or from about 5 minutes to about 15 minutes. In methods where fucose infusion is used in conjunction with a continuous feed schedule or with an extended feed schedule, the fucose infusion is daily or at least every other day, and about the same time of day to ensure that there is at least one fucose infusion per twenty four hour period. Fucose infusions occur, once initiated, daily or at least every other day from the onset of the feed media infusion until the end of the cell culture, or until it is desired to end feed media infusion into the cell culture or until it is desired to end fucose infusion.

In some preferred embodiments, the amount of fucose that is infused into the cell culture is the same amount each day, though greater or lesser amounts of fucose may be infused into the culture on different days. The amount of fucose that is infused is an amount effective to reduce the level of afucosylated species of the monoclonal antibody in the bioreactor-expressed preparation to a level that is desired. For example, in the biosimilar context, a desired level may be a level that approximates the level of afucosylated species present in the reference antibody preparation.

In examples, from about 0.05 g per liter of cell culture (g/L) to about 10 g per liter (g/L) of cell culture of fucose is infused per day, *e.g.,* as a bolus. In some embodiments of the invention, from about 0.05 g/L to about 5 g/L of fucose is infused per day, *e.g.,* as a bolus. In some examples, from about 0.1 g/L to about 10 g/L, from about 0.1 g/L to about 3 g/L of fucose, from about 0.1 g/L to about 2 g/L, from about 0.5 g/L to about 10 g/L of fucose, from about 1 g/L to about 10 g/L, from about 1 g/L to about 5 g/L, from about 1 g/L to about 4 g/L, from about 1 g/L to about 3 g/L, from about 1 g/L to about 2 g/L, from about 2 g/L to about 5 g/L, from about 2 g/L to about 4 g/L, from about 2 g/L to about 3 g/L, from about 3 g/L to about 5 g/L, from about 3 g/L to about 4 g/L, or from about 4 g/L to about 5 g/L of cell culture of fucose is infused per day, *e.g.,* as a bolus.

The cell culture can be a bioreactor cell culture, for example, a fermentation cell culture. In the embodiments of the invention, the cells are recombinant, and express a monoclonal antibody. In certain embodiments, the cells may be eukaryotic, with mammalian cells being most preferred. Non-limiting examples of mammalian cells that are suitable for expressing monoclonal antibodies in accordance with the methods described or exemplified herein include Chinese Hamster Ovary (CHO) cells, human embryonic kidney 293 (HEK293) cells, Sp2/0 cells, and NSO cells.

The methods are suitable for use with any monoclonal antibody expressed by such cells in a bioreactor. In some preferred embodiments, monoclonal antibodies specifically bind to tumor necrosis factor (TNF) alpha. Any monoclonal antibodies that specifically bind to TNF alpha can be used, including, but not limited to the antibodies described in U.S. Patent No. 6,090,382.

In some preferred embodiments, the antibody is a full length monoclonal antibody, including both variable and constant regions. The antibody may have a heavy chain constant region and/or a light chain constant region. The antibody may include a derivative or fragment or portion of a full-length antibody that retains the antigen-binding specificity, and also retains most or all of the affinity, of the full length antibody molecule. The antibody may include post-translational modifications (PTMs) or moieties, which may impact antibody activity or stability. The antibody may be methylated, acetylated, glycosylated, sulfated, phosphorylated, carboxylated, and/or amidated, and may include other moieties that are well known in the art.

Continuous feeding and extended feeding schemes may be employed to enhance the concentration of afucosylated species of monoclonal antibodies. The enhancement may be tempered by the infusion of fucose into the bioreactor cell culture. Nevertheless, infusion of fucose into the bioreactor cell culture may be used to inhibit the production of afucosylated antibody species generally, regardless of whether a continuous feed or extended feed scheme is employed, for example, as described and exemplified herein as suitable for reducing other undesired antibody variants and species in the antibody preparation. The infusion of fucose may also be used along with a bolus feed scheme (for infusion of the cell culture with feed media). Thus, according to this disclosure, the level of afucosylated species may be modulated.

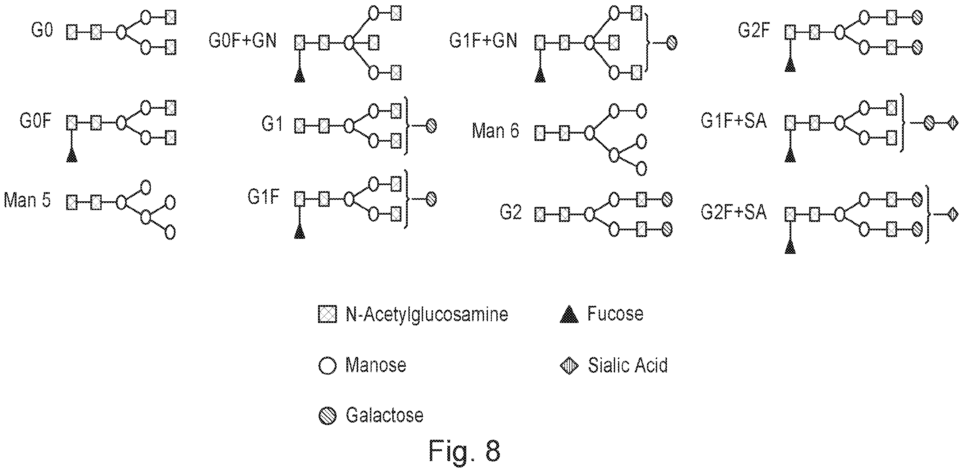

Afucosylated species may include monoclonal antibodies that contain any one or more of the GO glycan, G1a glycan, G1b glycan, G2 glycan, Man 3 glycan, Man 4 glycan, Man 5 glycan, Man 6 glycan, Man 7 glycan, Man 8 glycan, or Man 9 glycan (Fig. 8). The methods can be used to modulate the levels of monoclonal antibody species containing the GO glycan. The afucosylated species may include monoclonal antibodies that contain the GO glycan, G1 glycan, G2 glycan, Man 5 glycan and Man 6 glycan. In some embodiments, the afucosylated species contain the GO glycan and the Man 5 glycan. For anti-TNF monoclonal antibodies, the GO glycan may include from about 3% to about 7%, the G1 glycan may include from about 0.8% to about 2%, the Man 5 glycan may include from about 0.8% to about 1.8% of the monoclonal antibodies expressed in the bioreactor.

Fucose infusion may reduce the level of afucosylated species of the monoclonal antibody to about 20% or less of the total amount of monoclonal antibody in the preparation (the total amount of monoclonal antibody including the desired antibody molecule as well as species and variants thereof). Fucose infusion may reduce the level of afucosylated species to about 10% or less of the total amount of monoclonal antibody in the preparation. The total amount of monoclonal antibody includes the desired main monoclonal antibody as well as all variant species, including acidic species, basic species, afucosylated species, aggregates, high molecular weight species, fragments, and other antibody species. Fucose infusion may reduce the level of afucosylated species to about 9% or less, about 8% or less, about 7% or less, about 6% or less, about 5% or less, about 4% or less, about 3% or less, about 2% or less, or about 1% or less of the total amount of monoclonal antibody in the preparation. Fucose infusion may reduce the level of afucosylated species to from about 1% to about 20%, from about 1% to about 15%, from about 1% to about 12%, from about 1% to about 10%, from about 1% to about 8%, from about 1% to about 6%, from about 2% to about 16%, from about 2% to about 13%, from about 2% to about 10%, from about 2% to about 8%, from about 2% to about 6%, from about 3% to about 8%, from about 3% to about 6%, from about 3% to about 5%, from about 4% to about 18%, from about 4% to about 12%, from about 4% to about 8%, from about 4% to about 6%, from about 5% to about 15%, from about 5% to about 10%, from about 6% to about 18%, from about 6% to about 12%, from about 8% to about 15%, from about 8% to about 12%, or from about 10% to about 20% of the total amount of monoclonal antibody in the preparation.

It was observed that bolus infusion of feed media over the course of the cell culture produced a level of about 6-7% (of the total antibody preparation) afucosylated species of the antibody. About 5% of the total antibody preparation, or about 70-85% of the total afucosylated species, contained the GO glycan species. It was further observed that an extended feed or continuous feed (infusion of feed media) scheme, while reducing levels of high molecular weight species, acidic species, and fragments of the antibody, enhanced the level of afucosylated species to about 8.5-9.5% of the total antibody preparation. Under these feed schemes, about 6-7% of the total antibody preparation, or about 60-75% of the total afucosylated species, contained the GO glycan species.

Fucose infusion reduced both the overall level of afucosylated species and the level of GO glycan species of antibodies when coupled to an extended feed or continuous feed scheme. This reduction may be tailored to achieve the bolus-feed levels, or may be driven lower than the bolus-feed levels, if desired. Fucose infusion did not enhance the extended feed- or continuous feed-reduced levels of high molecular weight, acidic, or fragment species or variants of the antibodies.

Fucose infusion may reduce the level of total afucosylated species from about 1% to about 99% or more of the level of afucosylated antibody species produced in a cell culture in which no fucose infusion, or in which fucose was not infused substantially every day coinciding with the infusion of feed media. In some embodiments, fucose infusion may reduce the level of total afucosylated species from about 5% to about 80%, from about 5% to about 70%, from about 5% to about 60%, from about 5% to about 50%, from about 5% to about 40%, from about 5% to about 30%, from about 5% to about 20%, from about 10% to about 70%, from about 10% to about 60%, from about 10% to about 50%, from about 10% to about 40%, from about 10% to about 30%, from about 10% to about 20%, from about 20% to about 60%, from about 20% to about 50%, from about 20% to about 40%, from about 20% to about 30%, from about 30% to about 60%, from about 30% to about 50%, from about 30% to about 40%, from about 40% to about 70%, from about 40% to about 60%, from about 50% to about 80%, from about 50% to about 70%, or from about 50% to about 60% of the level of afucosylated antibody species produced in a cell culture in which no fucose infusion, or in which fucose was not infused substantially every day coinciding with the infusion of feed media. Fucose infusion may reduce the level of afucosylated species produced in a bolus-feed, extended feed, or continuous feed cell culture.

Fucose infusion may reduce the level of GO glycan species from about 1% to about 99% or more of the level of GO glycan antibody species produced in a cell culture in which no fucose infusion, or in which fucose was not infused substantially every day coinciding with the infusion of feed media. Fucose infusion may reduce the level of GO glycan species from about 5% to about 80%, from about 5% to about 70%, from about 5% to about 60%, from about 5% to about 50%, from about 5% to about 40%, from about 5% to about 30%, from about 5% to about 20%, from about 10% to about 70%, from about 10% to about 60%, from about 10% to about 50%, from about 10% to about 40%, from about 10% to about 30%, from about 10% to about 20%, from about 20% to about 60%, from about 20% to about 50%, from about 20% to about 40%, from about 20% to about 30%, from about 30% to about 60%, from about 30% to about 50%, from about 30% to about 40%, from about 40% to about 70%, from about 40% to about 60%, from about 50% to about 80%, from about 50% to about 70%, or from about 50% to about 60% of the level of GO glycan antibody species produced in a cell culture in which no fucose infusion, or in which fucose was not infused substantially every day coinciding with the infusion of feed media. Fucose infusion may reduce the level of GO glycan species produced in a bolus-feed, extended feed, or continuous feed cell culture.

Methods for enhancing afucosylated species of a monoclonal antibody recombinantly expressed in a bioreactor generally include culturing recombinant cells that express the monoclonal antibody in a bioreactor, then infusing the culture with feed media according to an extended feed or a continuous feed scheme, for example, the extended feed and continuous feed schemes described and exemplified herein, including those described above with respect to fucose infusion. Continuous feeding and extended feeding, without infusion of fucose, enhances the level of afucosylated species, particularly the GO glycan species, of the monoclonal antibody. Additionally, such continuous feeding and extended feeding, though the levels of afucosylated species are enhanced, reduce the levels of other antibody species such as high molecular weight species, acidic charge species, and fragments of the antibody, relative to a bolus feeding scheme. It is believed that as a consequence of the reduction of at least acidic charge species in this regard, the levels of the main antibody are enhanced. In other words, fewer acidic species are produced in the total antibody preparation and the loss in the percentage of acidic species is made up in the gain in the percentage of the main antibody in the preparation.

Any suitable feed media may be infused into the cell culture. Feed media generally are commercially available, and such commercially produced feed media are suitable for use in the methods described or exemplified herein. Feed media may include any media described or exemplified herein.

Infusion of feed media may begin on about day 1, about day 2, about day 3, about day 4, about day 5, about day 6, or about day 7 of a multi-day bioreactor cell culture, and continue thereafter until the conclusion of the cell culture. The cell culture maybe at least about 7 days, at least about 10 days, at least about 11 days, at least about 12 days, at least about 13 days, at least about 14 days, at least about 15 days, at least about 16 days, at least about 17 days, at least about 18 days, at least about 19 days, at least about 20 days, at least about 21 days, at least about 22 days, at least about 23 days, at least about 24 days, or more than 24 days. The cell culture can last for from about 10 days to about 21 days, from about 10 days to about 14 days, from about 10 days to about 12 days, or from about 11 days to about 13 days.

A continuous feed schedule involves infusing the cell culture with feed media continuously over a twenty four hour period. In some embodiments, the same amount/concentration of feed media that would ordinarily be infused as a bolus is infused into the culture, but that amount is apportioned to be substantially evenly infused over twenty four hours. A higher amount/concentration of feed media is infused over twenty four hours relative to the amount/concentration that would have been infused per bolus. A lesser amount/concentration of feed media is infused over twenty four hours relative to the amount/concentration that would have been infused per bolus. Thus, under a continuous feed schedule, feed media is infused into the cell culture constantly from the onset of the feed media infusion until the end of the cell culture, or until it is desired to end feed media infusion into the cell culture.

An extended feed schedule involves infusing the cell culture with feed media for a period longer than a bolus infusion, but not over a full twenty four hours. A bolus infusion includes an infusion over a period of from about 5 minutes to about one hour. Generally, a bolus infusion proceeds over a period of from about 5 minutes to about 15 minutes.

An extended feed schedule extends delivery of the feed media into the cell culture over a period of from about six hours to about twenty three hours. An extended feed schedule extends delivery of the feed media into the cell culture over a period of from about eight hours to about eighteen hours, from about twelve hours to about twenty two hours, from about twelve hours to about twenty hours, from about twelve hours to about eighteen hours, from about sixteen hours to about twenty two hours, from about sixteen hours to about twenty hours, from about sixteen hours to about eighteen hours, or from about seventeen hours to about nineteen hours. In some embodiments, an extended feed period of about eighteen hours is preferred. During the balance of time from the end of the extended feed period to the twenty four hour point, no feed media is infused into the cell culture. Thus, under an extended feed schedule, feed media is infused into the cell culture daily over the extended feed period (followed by a break until about the twenty four hour mark) from the onset of the feed media infusion until the end of the cell culture, or until it is desired to end feed media infusion into the cell culture.

Extended feeding or continuous feeding of the cell culture may enhance the level of total afucosylated species from about 1% to about 99% or more of the level of afucosylated antibody species produced in a cell culture fed via bolus feeding of feed media. Extended feeding or continuous feeding of the cell culture may enhance the level of total afucosylated species from about 5% to about 60%, from about 5% to about 50%, from about 5% to about 40%, from about 5% to about 30%, from about 5% to about 20%, from about 5% to about 15%, from about 5% to about 10%, from about 10% to about 50%, from about 10% to about 40%, from about 10% to about 30%, from about 10% to about 20%, from about 10% to about 15%, from about 20% to about 80%, from about 20% to about 70%, from about 20% to about 60%, from about 20% to about 50%, from about 20% to about 40%, from about 20% to about 30%, from about 30% to about 80%, from about 30% to about 70%, from about 30% to about 60%, from about 30% to about 50%, from about 30% to about 40%, from about 40% to about 90%, from about 40% to about 80%, from about 40% to about 70%, from about 40% to about 60%, from about 50% to about 90%, from about 50% to about 80%, from about 50% to about 70%, from about 50% to about 60%, from about 60% to about 90%, from about 60% to about 80%, from about 60% to about 70%, from about 70% to about 90%, or from about 75% to about 85% of the level of afucosylated antibody species produced in a cell culture fed via bolus feeding of feed media.

Extended feeding or continuous feeding of the cell culture may enhance the level of GO glycan antibody species from about 1% to about 99% or more of the level of GO glycan antibody species produced in a cell culture fed via bolus feeding of feed media. Extended feeding or continuous feeding of the cell culture may enhance the level of GO glycan antibody species from about 5% to about 60%, from about 5% to about 50%, from about 5% to about 40%, from about 5% to about 30%, from about 5% to about 20%, from about 5% to about 15%, from about 5% to about 10%, from about 10% to about 50%, from about 10% to about 40%, from about 10% to about 30%, from about 10% to about 20%, from about 10% to about 15%, from about 20% to about 80%, from about 20% to about 70%, from about 20% to about 60%, from about 20% to about 50%, from about 20% to about 40%, from about 20% to about 30%, from about 30% to about 80%, from about 30% to about 70%, from about 30% to about 60%, from about 30% to about 50%, from about 30% to about 40%, from about 40% to about 90%, from about 40% to about 80%, from about 40% to about 70%, from about 40% to about 60%, from about 50% to about 90%, from about 50% to about 80%, from about 50% to about 70%, from about 50% to about 60%, from about 60% to about 90%, from about 60% to about 80%, from about 60% to about 70%, from about 70% to about 90%, or from about 75% to about 85% of the level of GO glycan antibody species produced in a cell culture fed via bolus feeding of feed media.

Extended feeding or continuous feeding of the cell culture may enhance the level of total afucosylated species to from about 0.5% to about 20% of the total level of monoclonal antibody produced in a cell culture. Extended feeding or continuous feeding of the cell culture may enhance the level of total afucosylated species to from about 1% to about 15%, from about 1% to about 5%, from about 1% to about 7%, from about 1% to about 5%, from about 1% to about 3%, from about 2% to about 7%, from about 2% to about 10%, from about 2% to about 5%, from about 2% to about 4%, from about 3% to about 10%, from about 3% to about 9%, from about 3% to about 6%, from about 3% to about 5%, from about 4% to about 12%, from about 4% to about 10%, from about 4% to about 8%, from about 4% to about 7%, from about 4% to about 6%, from about 5% to about 17%, from about 5% to about 15%, from about 5% to about 13%, from about 5% to about 12%, from about 5% to about 11%, from about 5% to about 10%, from about 5% to about 9%, from about 5% to about 8%, from about 5% to about 7%, from about 5% to about 6%, from about 6% to about 20%, from about 6% to about 15%, from about 6% to about 13%, from about 6% to about 12%, from about 6% to about 11%, from about 6% to about 10%, from about 6% to about 9%, from about 7% to about 18%, from about 7% to about 15%, from about 7% to about 13%, from about 7% to about 12%, from about 7% to about 11%, from about 7% to about 10%, from about 7.5% to about 9.5%, from about 8% to about 15%, from about 8% to about 13%, from about 8% to about 12%, from about 8% to about 11, from about 8% to about 10%, from about 8% to about 9%, from about 8.5% to about 9.5%, from about 9% to about 15%, from about 9% to about 13%, from about 9% to about 12%, from about 9% to about 11%, from about 9% to about 10%, from about 10% to about 15%, from about 10% to about 13%, or from about 10% to about 12% of the total level of monoclonal antibodies produced in a cell culture. Extended feed or continuous feed cell culturing may enhance the level of afucosylated species relative to the levels of afucosylated species expressed by a bolus fed cell culture.

Extended feeding or continuous feeding of the cell culture may enhance the level of GO glycan species to from about 5% to about 15% of the total level of monoclonal antibody produced in a cell culture. Extended feeding or continuous feeding of the cell culture may enhance the level of GO glycan species to from about 5% to about 14%, from about 5% to about 13%, from about 5% to about 12%, from about 5% to about 11%, from about 5% to about 10%, from about 5% to about 9%, from about 5% to about 8%, from about 5% to about 7%, from about 5% to about 6%, from about 6% to about 15%, from about 6% to about 14%, from about 6% to about 13%, from about 6% to about 12%, from about 6% to about 11%, from about 6% to about 10%, from about 6% to about 9%, from about 6% to about 8%, from about 6% to about 7%, from about from about 7% to about 15%, from about 7% to about 13%, from about 7% to about 12%, from about 7% to about 11%, from about 7% to about 10%, from about 7% to about 9%, or from about 7% to about 8% of the total level of monoclonal antibodies produced in a cell culture. Extended feed or continuous feed cell culturing may enhance the level of GO glycan species relative to the levels of GO glycan species expressed in a bolus fed cell culture.

The following examples are provided to describe the invention in greater detail. They are intended to illustrate, not to limit, the invention.

### Example 1

### Base Feeding Strategy

These experiments were undertaken to develop a purification process that addresses the complex challenge of controlling the heterogeneity created during bioreactor production of therapeutic monoclonal antibodies. Upstream process development for the basal/feed media began with media screening experiments utilizing a scaled-down model of bench top bioreactors. The screening evaluated several basal media types crossed with several feed media types to determine an appropriate combination. Product quality and productivity were among the criteria used for media selection. Following the selection of basal/feed media, the product quality and productivity were monitored and modulated by altering process parameters and adding supplements.

Size-exclusion chromatography (SE-UPLC) was used to monitor antibody size variant distribution. The method was isocratic with a sodium phosphate running buffer, using a Waters Acquity UPLC BEH200 SEC column (1.7µm, 4.6x150mm). Peaks were monitored using absorbance at 280nm. Species eluting before the monomer peak were aggregates (high MW species) and peaks eluting after the monomer peak were degradants (low MW species).

Cation exchange HPLC (CEX-HPLC) was used to monitor charged species, including C-terminal variants, via weak cation exchange chromatography at a pH range of 5.6 to 8.0. Distinct peaks eluting after the main peak were considered basic species, and peaks eluting prior to the main peak were considered acidic species. Basic peaks contain C-terminal lysine and amidated proline variants. Modifications such as glycation and deamidation may be present in the acidic peaks.

For the base process, a basal medium that is chemically defined (CD) and devoid of animal components was selected. Media was selected if it contained enough nutrients to sustain cell growth for about four to five days and maintained a cell viability of ≥95%. The nutrients were selected to be balanced in such a way the waste and metabolites were within acceptable concentrations. Five basal media were screened for use in a base process (Table 1).

**Table 1. Basal Media Screened**

| **Media** | **Vendor** |
|---|---|
| CD CHO ACT | Thermo Fisher Scientific |
| CD OptiCHO | Thermo Fisher Scientific |
| CD FortiCHO | Thermo Fisher Scientific |
| BalanceCD CHO SLX A | Irvine Scientific |
| HyCell CHO | GE Healthcare |

For the base process, a feed medium that is chemically defined (CD) and devoid of animal components was selected. Medium was selected to sustain cell growth for four to five days and maintain cell viability of ≥95%. Nutrients were balanced in such a way that the waste and metabolites were within acceptable concentrations. Feed media that were screened for use in the base process are shown in Table 2.

**Table 2. Feed Media Screened**

| **Media** | **Vendor** |
|---|---|
| Cell Boost 5 | GE Healthcare |
| BalanceCD CHO 1 | Irvine Scientific |
| BalanceCD CHO 2 | Irvine Scientific |
| BalanceCD CHO 3 | Irvine Scientific |
| CD Efficient Feed A | Thermo Fisher Scientific |
| CD Efficient Feed B | Thermo Fisher Scientific |
| CD Efficient Feed C | Thermo Fisher Scientific |

A design study was conducted to evaluate the relationship between the basal and feed media (Tables 3 and 4). The goal of this study was to develop a base process that incorporates a basal and feed media that provides great growth, viability, and adequate antibody expression. Once the criteria were met, a basal and a feed media were chosen to be the base process.

**Table 3. Basal and Feed media screening, test #1.**

| **Basal Medium** | **Feed Medium** | **Feed Volume** | **Feed Days** |
|---|---|---|---|
| CD CHO AGT | BalanCD CHO 1 | 10% | 4,6,8 |
| BalanCD CHO SLX A | BalanCD CHO 1 | 10% | 4,6,8 |
| CD OptiCHO | CHO Efficient C | 10% | 4,6,8 |
| BalanCD CHO SLX A | BalanCD CHO 1 | 10% | 4,6,8 |
| HyCell | BalanCD CHO 1 | 10% | 4,6,8 |
| BalanCD CHO SLX A | Cell Boost | 5% | 4,6,8 |
| BalanCD CHO SLX A | CHO Efficient C | 10% | 4,6,8 |
| CD OptiCHO | Cell Boost | 5% | 4,6,8 |
| HyCell | Cell Boost | 5% | 4,6,8 |
| CD OptiCHO | BalanCD CHO 1 | 10% | 4,6,8 |
| CD CHO AGT | CHO Efficient C | 10% | 4,6,8 |
| CD OptiCHO | CHO Efficient C | 10% | 4,6,8 |
| HyCell | Cell Boost | 5% | 4,6,8 |
| HyCell | BalanCD CHO 1 | 10% | 4,6,8 |
| CD CHO AGT | Cell Boost | 5% | 4,6,8 |
| CD CHO AGT | Cell Boost | 5% | 4,6,8 |
| CD CHO AGT | BalanCD CHO 1 | 10% | 4,6,8 |
| CD OptiCHO | Cell Boost | 5% | 4,6,8 |
| BalanCD CHO SLX A | Cell Boost | 5% | 4,6,8 |
| HyCell | CHO Efficient C | 10% | 4,6,8 |
| BalanCD CHO SLX A | CHO Efficient C | 10% | 4,6,8 |
| HyCell | CHO Efficient C | 10% | 4,6,8 |
| CD OptiCHO | BalanCD CHO 1 | 10% | 4,6,8 |
| CD CHO AGT | CHO Efficient C | 10% | 4,6,8 |

**Table 4. Basal and Feed media screening, test #2.**

| **Basal Medium** | **Feed Medium** | **Feed Volume** | **Feed Days** |
|---|---|---|---|
| CD FortiCHO | BalanCD CHO 3 | 10% | 1,3,5 |
| BalanCD SLX A | CHO Efficient B | 10% | 2,4,6,8 |
| CD FortiCHO | CHO Efficient C | 10% | 2,4,6,8 |
| HyCell | CHO Efficient A+B | 10% | 2,4,6,8 |
| CD FortiCHO | CHO Efficient A+B | 10% | 2,4,6,8 |
| HyCell | CHO Efficient B | 10% | 2,4,6,8 |
| CD CHO AGT | BalanCD CHO 3 | 10% | 1,3,5 |
| CD FortiCHO | Cell Boost 5 | 5% | 4,6,8,10,12 |
| BalanCD SLX A | BalanCD CHO 3 | 10% | 1,3,5 |
| BalanCD SLX A | CHO Efficient C | 10% | 2,4,6,8 |
| CD CHO AGT | CHO Efficient A+B | 10% | 2,4,6,8 |
| CD OptiCHO | BalanCD CHO 2 | 10% | 1,3,5 |
| CD OptiCHO | CHO Efficient B | 10% | 2,4,6,8 |
| CD OptiCHO | CHO Efficient C | 10% | 2,4,6,8 |
| HyCell | BalanCD CHO 1 | 10% | 1,3,5 |
| CD OptiCHO | Cell Boost 5 | 5% | 4,6,8,10,12 |
| BalanCD SLX A | Cell Boost 5 | 5% | 4,6,8,10,12 |
| CD FortiCHO | CHO Efficient A | 10% | 2,4,6,8 |
| CD CHO AGT | CHO Efficient B | 10% | 2,4,6,8 |
| CD OptiCHO | BalanCD CHO 1 | 10% | 1,3,5 |
| BalanCD SLX A | CHO Efficient A | 10% | 2,4,6,8 |
| CD FortiCHO | BalanCD CHO 2 | 10% | 1,3,5 |
| HyCell | BalanCD CHO 3 | 10% | 1,3,5 |
| HyCell | CHO Efficient C | 10% | 2,4,6,8 |
| CD CHO AGT | CHO Efficient C | 10% | 2,4,6,8 |
| CD OptiCHO | CHO Efficient C | 10% | 2,4,6,8 |
| BalanCD SLX A | CHO Efficient A+B | 10% | 2,4,6,8 |
| HyCell | CHO Efficient C | 10% | 2,4,6,8 |
| HyCell | Cell Boost 5 | 10% | 2,4,6,8 |
| CD OptiCHO | CHO Efficient A+B | 10% | 2,4,6,8 |
| HyCell | Cell Boost 5 | 10% | 1,3,5 |
| HyCell | Cell Boost 5 | 10% | 1,3,5 |
| BalanCD SLX A | BalanCD CHO 1 | 10% | 1,3,5 |
| CD FortiCHO | BalanCD CHO 1 | 10% | 1,3,5 |
| HyCell | BalanCD CHO 1 | 10% | 1,3,5 |
| BalanCD SLX A | BalanCD CHO 2 | 10% | 1,3,5 |
| CD CHO AGT | BalanCD CHO 1 | 10% | 1,3,5 |
| CD OptiCHO | CHO Efficient A | 10% | 2,4,6,8 |
| HyCell | Cell Boost 5 | 5% | 4,6,8,10,12 |
| CD FortiCHO | CHO Efficient B | 10% | 2,4,6,8 |
| CD CHO AGT | CHO Efficient A | 10% | 2,4,6,8 |
| HyCell | Cell Boost | 10% | 2,4,6,8 |
| CD OptiCHO | BalanCD CHO 3 | 10% | 1,3,5 |
| CD OptiCHO | BalanCD CHO 1 | 10% | 1,3,5 |
| HyCell | CHO Efficient A | 10% | 2,4,6,8 |
| CD CHO AGT | BalanCD CHO 2 | 10% | 1,3,5 |
| HyCell | BalanCD CHO 2 | 10% | 1,3,5 |
| CD CHO AGT | Cell Boost | 5% | 4,6,8,10,12 |

From the design study, as well as experiments at the shake flask and bioreactor scale, the base line process was determined. HyCell was selected as the basal medium and CD Efficient Feed C was selected as the feed medium. The feed medium was supplemented to the culture starting day 4, and then the culture was additionally fed every other day thereafter for a total of four additions ("Process A"). The typical process trends for this baseline process are shown in Figs. 1A (viable cell density), IB (% viability), 1C (titer), ID (acid species), IE (intact antibody), and IF (GO afucosylated species).

### Example 2

### Feeding Strategy

It was determined that some attributes of the monoclonal antibody preparation can be modulated in a way so as to improve certain critical quality attributes (CQAs). This modulation was achieved by altering the way the nutrient feed was introduced into the cell culture. In Example 1 above, the base process utilized a bolus feed, predicated on a specific cell density, with the feed added to the culture every other day beginning on day 4 of the bioreactor culture. Bolus feeding included nutrient addition to the cell culture over a period of about 15 total minutes every other day. But it was determined thereafter that the feed strategy impacted the levels of certain aspects of the protein preparation, including the level of high MW species, antibody fragments, and charge variants.

During the cell culturing process, it was observed that the feeding strategy can modulate certain critical quality attributes (e.g., charge heterogeneity, fragmentation, aggregation etc.). The bioreactor feed processes tested were categorized as bolus feeding: 5-30 min of feed, every other day beginning on day 4(Process A), extended feeding: 10-18 hours of feed, every day, beginning on day 4 (Process B), or continuous feeding: 24 hours of feed per day, every day beginning on day 4 (Process C). It was observed that the addition of feed by either extended periods or by continuous means can improve some quality attributes of the expressed antibody protein (Figs. 2A-2F), and it was further observed that such feed variations were beneficial to the biosimilar antibody batch because the resultant protein preparation more closely matched the reference product, lessening the need for downstream processing.

Of note, the overall amount of nutrients fed into the bioreactor was not changed depending on whether Process A, Process B, or Process C was employed. Rather, the time and distribution of the amount was altered accordingly among the feed processes. The total amount of feed stays constant no matter what strategy is employed. Feeding according to Process B and Process C produced several beneficial results: increased titer, reduced acidic charge variant accumulation, higher intact mAb (lower fragments), longer culture longevity and reduced aggregation (Figs. 2A-2F).

### Example 3

### aFucosylated species control

Despite the beneficial results observed for bioreactor feeding according to Process B or Process C (relative to Process A), one consequence of the process change was an increase in the level of afucosylated species and, in particular, the GO species.

Glycosylation of an antibody (e.g., IgG isotype) impacts the effector function (e.g., Antibody-Dependent cell cytotoxicity (ADCC)) of the molecule. Therapeutic antibodies that have no fucose or low fucose exhibit an increased binding to activating FcyRIIIA and trigger a strong ADCC response relative to their highly-fucosylated counterparts. In terms of biosimilar development, afucosylated variant levels have implications for how the biosimilar antibody will behave relative to the reference antibody. For example, if an expressed biosimilar has increased afucosylated content relative to the reference product having a lower content, it is believed that this may result in the biosimilar triggering a stronger ADCC response *in vivo* relative to the reference product. Such differences may cause the antibody to lose status as a biosimilar. More broadly speaking, enhancing the afucosylated content in any therapeutic antibody preparation (biosimilar or not) may induce an undesired ADCC response *in vivo.* Thus, control of the content of afucosylated variant antibodies produced in a bioreactor is desirable in certain therapeutic contexts.

Accordingly, altering the feed strategy to reduce or eliminate certain undesirable species of antibody variants may at the same time produce yet other undesirable species of antibody variants; essentially, solve one problem and create another problem. Thus, experiments according to this Example were undertaken to attain the best of both worlds - control of undesirable species and control of afucosylated variants. Note that enhancement of afucosylated variants may be desired in some contexts.

Process C was found to enhance desirable attributes of the antibody preparation, but also to enhance the level of G0 afucosylated antibody variants. In order to reduce the afucosylated species, several experiments further varying the feeding process were undertaken. In one such series of experiments, the addition of fucose to the bioreactor was assessed.

It was observed that, according to Process C, lowering the afucosylated species, especially G0, can be achieved by supplementing L-Fucose to the cell culture medium at a concentration of from about 1 g/L to about 3 g/L. The route of administration was also observed to be relevant.

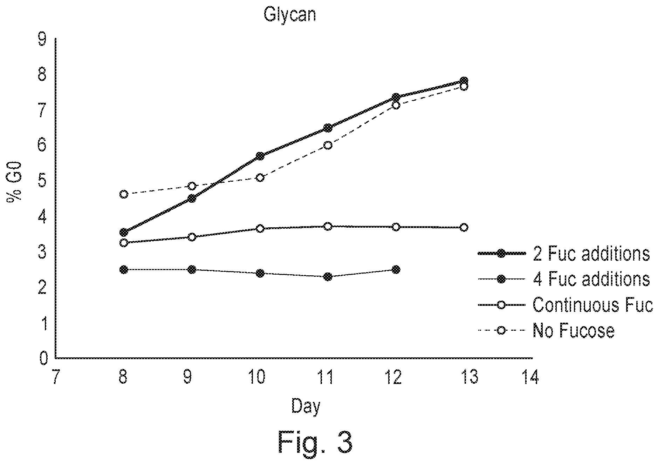

It was observed that the best results were observed when fucose was added daily or every other day. It was also observed that feeding of fucose (one or two bolus additions) in the early stages of the cell culture did not inhibit the increase of the afucosylated species, particularly the GO species (Fig. 3).

In one experiment, fucose addition occurred on day 4 and 6 only (2 Fucose additions). It was that fucose addition had an initial GO lowering effect, but then GO levels increased significantly, with GO levels essentially matching the levels observed for cultures in which supplemental fucose was not added to the culture. Further experimentation revealed that fucose must be included throughout the cell culture in order to reduce the afucosylated species. Bolus additions of fucose, either daily or every other day, was found to lower GO species levels to desired amounts.

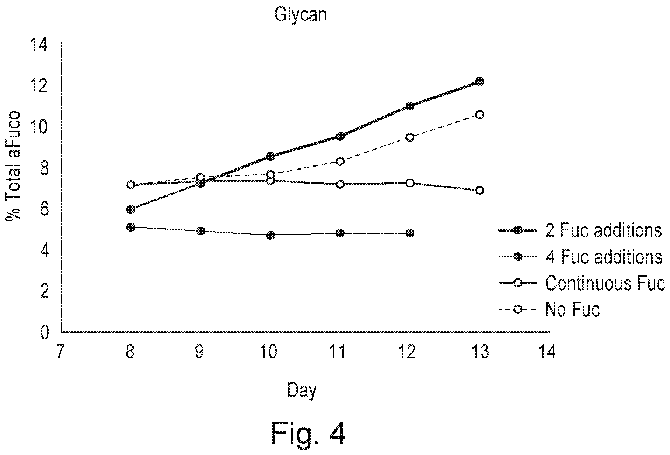

Looking at the total afucosylated species (beyond GO species), the same trend was observed (Fig. 4). Early fucose addition to the culture alone, and no infusion of fucose to the cell culture yielded an overall higher amount of afucosylated species in contrast to the more continuous addition of fucose to the cell culture. As seen in (Fig. 4), the overall afucosylated fucose levels remained the same throughout the process. Fucose needs to be added throughout the culture duration, because if only added in the first few days, the lowering of afucosylated species that occurs subsides and the level of afucosylated species increased over time, as shown in Fig. 3. Most preferred is when the fucose is added in four bolus additions without continuous feed addition.

### Example 4

### Summary

These data are indicative of an upstream process that is capable of modulating quality control attributes of a recombinant antibody expressed in a bioreactor. The process combines an altered bioreactor feed process coupled with the continual addition of fucose. With the process of an extended/continuous feeding strategy and the supplementation of fucose, the combination yields lower product related impurities (e.g., acidic species, GO species).

### EQUIVALENTS

The details of one or more embodiments of the invention are set forth in the accompanying description above. Although any methods and materials similar or equivalent to those described herein can be used in the practice or testing of the present invention, the preferred methods and materials are now described.

## Claims

1. A method for reducing afucosylated species of a monoclonal antibody recombinantly expressed in a bioreactor, comprising culturing recombinant cells that express the monoclonal antibody in a bioreactor, beginning on the first, second, third, or fourth day of the culture, and continuing every other day until the conclusion of the culture, infusing the culture with from about 0.5 g/L to about 5 g/L of fucose such that afucosylated species of the monoclonal antibody expressed by the cells are reduced.

2. The method according to claim 1, wherein the monoclonal antibody specifically binds to tumor necrosis factor (TNF) alpha.

3. The method according to any one of claims 1 to 2, wherein the recombinant cells comprise mammalian cells, Chinese Hamster Ovary cells, HEK293 cells or Sp2/0 cells.

4. The method according to any one of claims 1 to 3, wherein the method comprises infusing the culture with from about 1 g/L to about 5 g/L, 4g/L, 3 g/L or 2 g/L of fucose.

5. The method according to any one of claims 1 to 3, wherein the method comprises infusing the culture with from about 2 g/L to about 4 g/L of fucose.

6. The method according to any one of claims 1 to 4, wherein the fucose is infused in a bolus.

7. The method according to any one of claims 1 to 6, wherein the culture is a continuous-feed culture in which the culture is further infused with a feed media continuously over a twenty four hour period beginning on the second, third, or fourth day of the culture, and continuing every day until the conclusion of the culture.

8. The method according to any one of claims 1 to 6, wherein the culture is an extended-feed culture in which the culture is further infused with a feed media continuously over a period of from about eighteen to about twenty hours beginning on the second, third, or fourth day of the culture, and continuing every day until the conclusion of the culture.

9. The method according to any one of claims 1 to 8, wherein the afucosylated species comprise the GO glycan, G1a glycan, G1b glycan, G2 glycan, Man 3 glycan, Man 4 glycan, Man 5 glycan, Man 6 glycan, Man 7 glycan, Man 8 glycan, or Man 9 glycan.

10. The method according to any one of claims 1 to 9, wherein the afucosylated species comprises the GO glycan.

11. The method according to any one of claims 1 to 10, wherein the afucosylated species of the monoclonal antibody are reduced to about 10% or less of the total amount of monoclonal antibody expressed by the cells.

12. The method according to any one of claims 1 to 11, wherein the afucosylated species of the monoclonal antibody are reduced to from about 2% to about 10% of the total amount of monoclonal antibody expressed by the cells.

13. The method according to any one of claims 1 to 10, wherein the afucosylated species of the monoclonal antibody are reduced to about 6% or less of the total amount of monoclonal antibody expressed by the cells.

14. The method according to any one of claims 1 to 13, wherein the afucosylated species of the monoclonal antibody are reduced to from about 2% to about 6% of the total amount of monoclonal antibody expressed by the cells.

15. The method according to any one of claims 1 to 14, wherein the method comprises culturing recombinant cells that express the monoclonal antibody in a bioreactor for at least twelve days.

## Patentansprüche

1. Verfahren zum Reduzieren afucosylierter Spezies eines monoklonalen Antikörpers, der rekombinant in einem Bioreaktor exprimiert wird, das das Kultivieren von den monoklonalen Antikörper exprimierenden rekombinanten Zellen in einem Bioreaktor beinhaltet, wobei die Kultur, beginnend am ersten, zweiten, dritten oder vierten Tag der Kultur und fortlaufend jeden zweiten Tag bis zum Abschluss der Kultur, mit etwa 0,5 g/l bis etwa 5 g/l Fucose infundiert wird, so dass afucosylierte Spezies des von den Zellen exprimierten monoklonalen Antikörpers reduziert werden.

2. Verfahren nach Anspruch 1, wobei sich der monoklonale Antikörper spezifisch an Tumornekrosefaktor-(TNF)-alpha bindet.

3. Verfahren nach einem der Ansprüche 1 bis 2, wobei die rekombinanten Zellen Säugetierzellen, Ovarialzellen des Chinesischen Hamsters, HEK293-Zellen oder Sp2/0-Zellen umfassen.

4. Verfahren nach einem der Ansprüche 1 bis 3, wobei das Verfahren das Infundieren der Kultur mit etwa 1 g/l bis etwa 5 g/l, 4 g/l, 3 g/l oder 2 g/l Fucose beinhaltet.

5. Verfahren nach einem der Ansprüche 1 bis 3, wobei das Verfahren das Infundieren der Kultur mit etwa 2 g/l bis etwa 4 g/l Fucose beinhaltet.

6. Verfahren nach einem der Ansprüche 1 bis 4, wobei die Fucose in einem Bolus infundiert wird.

7. Verfahren nach einem der Ansprüche 1 bis 6, wobei die Kultur eine Kultur mit kontinuierlicher Zufuhr ist, wobei die Kultur ferner kontinuierlich über einen Zeitraum von vierundzwanzig Stunden, beginnend am zweiten, dritten oder vierten Tag der Kultur und fortlaufend jeden Tag bis zum Abschluss der Kultur, mit einem Zufuhrmedium infundiert wird.

8. Verfahren nach einem der Ansprüche 1 bis 6, wobei die Kultur eine Kultur mit verlängerter Zufuhr ist, wobei die Kultur ferner kontinuierlich über einen Zeitraum von etwa achtzehn bis etwa zwanzig Stunden, beginnend am zweiten, dritten oder vierten Tag der Kultur und fortlaufend jeden Tag bis zum Abschluss der Kultur, mit einem Zufuhrmedium infundiert wird.

9. Verfahren nach einem der Ansprüche 1 bis 8, wobei die afucosylierten Spezies das G0-Glycan, G1a-Glycan, G1b-Glycan, G2-Glycan, Man3-Glycan, Man4-Glycan, Man5-Glycan, Man6-Glycan, Man7-Glycan, Man8-Glycan oder Man9-Glycan umfassen.

10. Verfahren nach einem der Ansprüche 1 bis 9, wobei die afucosylierte Spezies das G0-Glykan umfasst.

11. Verfahren nach einem der Ansprüche 1 bis 10, wobei die afucosylierten Spezies des monoklonalen Antikörpers auf etwa 10 % oder weniger der Gesamtmenge des von den Zellen exprimierten monoklonalen Antikörpers reduziert werden.

12. Verfahren nach einem der Ansprüche 1 bis 11, wobei die afucosylierten Spezies des monoklonalen Antikörpers auf etwa 2 % bis etwa 10 % der Gesamtmenge des von den Zellen exprimierten monoklonalen Antikörpers reduziert werden.

13. Verfahren nach einem der Ansprüche 1 bis 10, wobei die afucosylierten Spezies des monoklonalen Antikörpers auf etwa 6 % oder weniger der Gesamtmenge des von den Zellen exprimierten monoklonalen Antikörpers reduziert werden.

14. Verfahren nach einem der Ansprüche 1 bis 13, wobei die afucosylierten Spezies des monoklonalen Antikörpers auf etwa 2 % bis etwa 6 % der Gesamtmenge des von den Zellen exprimierten monoklonalen Antikörpers reduziert werden.

15. Verfahren nach einem der Ansprüche 1 bis 14, wobei das Verfahren das Kultivieren rekombinanter Zellen, die den monoklonalen Antikörper exprimieren, in einem Bioreaktor für wenigstens zwölf Tage beinhaltet.

## Revendications

1. Procédé pour la réduction d'espèces afucosylées d'un anticorps monoclonal exprimé par voie de recombinaison dans un bioréacteur, comprenant la culture de cellules recombinées qui expriment l'anticorps monoclonal dans un bioréacteur, à partir du premier, deuxième, troisième ou quatrième jour de la culture et ensuite tous les deux jours jusqu'à la fin de la culture, l'infusion de la culture avec d'environ 0,5 g/l à environ 5 g/l de fucose de façon telle que les espèces afucosylées de l'anticorps monoclonal exprimé par les cellules sont réduites.

2. Procédé selon la revendication 1, dans lequel l'anticorps monoclonal se lie spécifiquement au facteur de nécrose tumorale (TNF) alpha.

3. Procédé selon l'une quelconque des revendications 1 à 2, dans lequel les cellules recombinées comprennent des cellules de mammifère, des cellules ovariennes de hamster chinois, des cellules HEK293 ou des cellules Sp2/0.

4. Procédé selon l'une quelconque des revendications 1 à 3, le procédé comprenant l'infusion de la culture avec d'environ 1 g/l à environ 5 g/l, 4 g/l, 3 g/l ou 2 g/l de fucose.

5. Procédé selon l'une quelconque des revendications 1 à 3, le procédé comprenant l'infusion de la culture avec d'environ 2 g/l à environ 4 g/l de fucose.

6. Procédé selon l'une quelconque des revendications 1 à 4, dans lequel le fucose est infusé en un bolus.

7. Procédé selon l'une quelconque des revendications 1 à 6, dans lequel la culture est une culture à alimentation continue dans laquelle la culture est encore infusée avec un milieu d'alimentation en continu sur une durée de vingt-quatre heures à partir du deuxième, troisième ou quatrième jour de la culture et ensuite chaque jour jusqu'à la fin de la culture.

8. Procédé selon l'une quelconque des revendications 1 à 6, dans lequel la culture est une culture à alimentation prolongée dans laquelle la culture est encore infusée avec un milieu d'alimentation en continu sur une durée d'environ dix-huit à environ vingt heures à partir du deuxième, troisième ou quatrième jour de la culture et ensuite chaque jour jusqu'à la fin de la culture.

9. Procédé selon l'une quelconque des revendications 1 à 8, dans lequel les espèces afucosylées comprennent le glycane G0, le glycane G1a, le glycane G1b, le glycane G2, le glycane Man 3, le glycane Man 4, le glycane Man 5, le glycane Man 6, le glycane Man 7, le glycane Man 8 ou le glycane Man 9.

10. Procédé selon l'une quelconque des revendications 1 à 9, dans lequel les espèces afucosylées comprennent le glycane G0.

11. Procédé selon l'une quelconque des revendications 1 à 10, dans lequel les espèces afucosylées de l'anticorps monoclonal sont réduites à environ 10 % ou moins de la quantité totale d'anticorps monoclonal exprimé par les cellules.

12. Procédé selon l'une quelconque des revendications 1 à 11, dans lequel les espèces afucosylées de l'anticorps monoclonal sont réduites à d'environ 2 % à environ 10 % de la quantité totale d'anticorps monoclonal exprimé par les cellules.

13. Procédé selon l'une quelconque des revendications 1 à 10, dans lequel les espèces afucosylées de l'anticorps monoclonal sont réduites à environ 6 % ou moins de la quantité totale d'anticorps monoclonal exprimé par les cellules.

14. Procédé selon l'une quelconque des revendications 1 à 13, dans lequel les espèces afucosylées de l'anticorps monoclonal sont réduites à d'environ 2 % à environ 6 % de la quantité totale d'anticorps monoclonal exprimé par les cellules.

15. Procédé selon l'une quelconque des revendications 1 à 14, le procédé comprenant la culture de cellules recombinées qui expriment l'anticorps monoclonal dans un bioréacteur pendant au moins douze jours.
